Europäisches Patentamt

European Patent Office

Office européen des brevets

(11) Veröffentlichungsnummer: **0 025 035**
**B1**

(12) **EUROPÄISCHE PATENTSCHRIFT**

(45) Veröffentlichungstag der Patentschrift: **28.08.85**

(21) Anmeldenummer: **80900032.6**

(22) Anmeldetag: **12.12.79**

(86) Internationale Anmeldenummer:
**PCT/CH79/00159**

(87) Internationale Veröffentlichungsnummer:
**WO 80/01955 18.09.80 Gazette 80/21**

(51) Int. Cl.⁴: **G 01 N 33/36, G 01 B 7/12**

(54) **VERFAHREN ZUR FESTSTELLUNG UND AUSWERTUNG VON FEHLERN IN GARNEN.**

(30) Priorität: **16.03.79 CH 2491/79**

(43) Veröffentlichungstag der Anmeldung:
**18.03.81 Patentblatt 81/11**

(45) Bekanntmachung des Hinweises auf die
Patenterteilung:
**28.08.85 Patentblatt 85/35**

(84) Benannte Vertragsstaaten:
**FR**

(56) Entgegenhaltungen:
**CH-B- 439 796**
**DE-C-1 209 777**
**FR-A-1 453 773**
**FR-A-1 584 684**
**GB-A-1 011 761**
**US-A-3 731 069**

(73) Patentinhaber: **ZELLWEGER USTER AG**
**Wilstrasse 11**
**CH-8610 Uster (CH)**

(72) Erfinder: **AEMMER, Peter F.**
**Bäumliacherstrasse 36**
**CH-8907 Wettswil a/A. (CH)**

(74) Vertreter: **Armengaud, Alain**
**Cabinet ARMENGAUD AINE 3 Avenue Bugeaud**
**F-75116 Paris (FR)**

Courier Press, Leamington Spa, England.

**Beschreibung**

Die Erfindung betrifft ein Verfahren zur Festellung und Auswertung von Fehlern in Garnen.

Die Qualität von Garnen ist um so besser und der dafür erzielbare Preis um so höher, je gleichmässiger ein Garn in Bezug auf Schwankungen seines Querschnittes bzw. Durchmessers ist. Mittels bekannter Bewertungsverfahren mit Hilfe von Garntafeln oder elektronischer Geräte wird der subjektive Eindruck der Garnqualität messbar gemacht. Auf Grund solcher messbarer Qualitätsmerkmale werden Garnreingungsanlagen gezielt sur Qualitätsverbesserung eingesetzt. Sie scheiden Garnfehler, die vorgegebene Grenzwerte eines oder mehrerer Qualitätsmerkmale überschreiten, während eine Umspulprozesses aus dem Garn und ersetzten den Garnfehler durch einen Knoten.

In der Praxis hat es sich eingebürgert, neben den statistisch verteilten Variationen des Garnquerschnittes bzw. Durchmessers über die ganze Länge eine Garnes die sporadisch auftretenden deutlichen Unregelmässigkeiten gesondert zu betrachten. Man unterscheidet dabei zwischen Verdickungen von etwa einem Millimeter bis einigen Zentimetern (Nissen, Schlonzen), längeren Dickstellen von einigen Zentimetern bis etwa 50 Zentimeter und mehr (Doppelfäden, Schleicher) und Dünnstellen von einigen Zentimetern bis mehreren Metern Länge.

Der Subjektive Eindruck, den die oben beschriebenen sporadischen Garnfehler sowohl in der Dimension Querschnitt bzw. Durchmesser als auch in der Dimension Länge hervorrufen, ist im wesentlichen derart, dass Abweichungen bezüglich der Sollwerte angenähert im logarithmischen Massstab erkannt werden.

Dieser annähernd logarithmische Zusammenhand zwischen subjektiver Empfindung und objektiver Erscheinungsform von sporadisch auftretenden Garnfehlern ist bekannt (siehe z.B. CH—A—439796 und FR—A—1453773). Das kommt beispielsweise in der üblichen Praxis der Darstellung und Bewertung von Garnfehlern in Fehlerklassen zum Ausdruck. Trotzdem sind bisher auf dem Gebiet der Garnfehleranalyse und Garnreinigung keine Verfahren bzw. Geräte bekannt geworden, welche den genannten logarithmischen Zusammenhang und das verhältnismässig seltene Auftreten von Garnfehlern gezielt zur technischen Vereinfachung oder zur Erhöhung der Leistungsfähigkeit solcher Geräte ausnützen. Die bisher eingesetzte Analogtechnik ermöglichte eine Signalanalyse mit linearen Filterelementen und nicht-linearen Diskriminatoren, mit denen ein Minimum an Schaltungsaufwand möglich war.

Durch den Uebergang zu digitaler Signalverarbeitung, unter Verwendung von Mikroprozessoren, Prozessorelementen und adressierbaren Speichern müssen andere Ueberlegungen angestellt werden. Eine blosse Nachbildung der herkömmlichen Analogtechnik mit Mitteln der digitalen Signalverarbeitung führt nicht zu zweckmässigen Lösungen, da die Digitaltechnik grundsätzlich anderen Möglichkeiten und Beschränkungen unterliegt als die bekannte Analogtechnik.

Des Ziel des nachfolgend beschriebenen Verfahrens besteht darin, dass der an sich bekannte angenähert logarithmische Zusammenhang zwischen subjektiver Fehlerempfindung und tatsächlicher Gestalt solcher Fehler absichtlich zur Erhöhung des Nutseffektes bzw. Verminderung des Aufwandes und damit der Kosten der elektronischen Anlage zur Reinigung von Garnen und zur Bewertung von Garnfehlern benütz wird.

Das erfindungsgemässe Verfahren ist besonders für Geräte der Digitaltechnik von Vorteil.

Die Erfindung beruht au den vorstehend gemachten Ueberlegungen und betrifft ein Verfahren zur Festellung und Auswertung von Fehlern in Garnen, bei dem:

— ein die Dicke des Garns als Funktion des Ortes repräsentierendes Messsignal erzeugt wird,
— diskrete aufeinanderfolgende Werte des Messsignals in logarithmierte und digitalisierte Zahlenwerte umgesetzt werden,
— die Zahlenwerte vor dem Vergleich zwischengespeichert werken und die Auswertung anhand dieser zwischengespeicherten Zahlenwerte entkoppelt von der Speicherung dieser Werte durchgeführt wird, indem,

a) die Länge des Garnabschnitts, über die eine Uberschreitung der Schwellwerte durch aufeinanderfolgend abgespeicherte Zahlenwerte stattfindet, mit vorgegebenen Sektionen unterschiedlicher Längen verglichen wird, und,

b) die Anzahl der zur Auswertung des Fehlers eines fehlerbehafteten Garnabschnitts herangezogenen zwischengespeicherten Zahlenwerte diese Abschnitts in Abhängigkeit vom Ergebnis des Vergleichs der Länge des fehlerbehafteten Garnabschnitts mit der Länge der vorgegebenen Sektionen gewählt wird, wobei die ausgewertete Anzahl von Zahlenwerten mit zunehmender Länge des Garnabschnitts reduziert wird.

In weiterer Ausgestaltung zeichnet sich das erfindungsgemässe Verfahren dadurch aus, dass das vom Garnsensor abgegebene Signal vor seiner digitalen Weiterverarbeitung durch Logarithmierung komprimiert und dadurch auf eine minimal benötige Wortlänge reduziert wird.

In der digitalen Signalverarbeitungstechnik werden in der Praxis zu diskreten Zeitpunkten abgetastete Signalwerte mittels algebraischer Algorithmen verarbeitet. Die einzelen Signalwerte und davon abgeleitete Grössen werden wortweise behandelt. Der Aufwand und damit die Kosten einer digitalen Signalverarbeitung hangen nun wesentlich von der für die Darstellung der Signale benötigten Wortlänge ab. Verfahren, welche mit kürzeren Wortlängen auskommen, erbringen somit einen wirtschaftlichen Vorteil gegenüber solchen, die eine Darstellung derselben Signale durch grössere Wortlängen benötigen.

Bekannte Verfahren der Garnanalyse und Garnreinigung mittels digital arbeitender Geräte erfassen Garnquerschnitt oder Durchmesser mittels Sensoren mit analogem Signalausgang. Nach einer eventuell nötigen Vorverarbeitung des analogen Signal mit Elementen der Analogtechnik (z.B. Tiefpassfilter, Driftkompensation u.a.) wird das Analogsignal einer A/D-Wandlung unterworfen. Ein solches Verfahren ist aus dem aus Dokument US—A—3737069 bekannt.

Das Verfahren gemäss einer besonderen Ausgestaltung der Erfindung unterscheidet sich nun von dieser herkömmlichen Technik dadurch, dass vor oder in einem der ersten Schritte der digitalen Signalverarbeitung eine Umwandlung der Signalwerte vom linearen zum mindestens angenähert logarithmischen Massstab vorgenommen wird. Damit wird zudem der weitere Vorteil erreicht, dass die technisch bedingte Auflösungsgrenze immer proportional zum jeweiligen subjektiven Qualitätsempfinden ist.

Wenn man davon ausgeht, dass eine digitales Gerät zur elektronischen Reinigung von Garnen und zur Bewertung von Garnfehlern aus einem Messwandler, einer Vorverarbeitungsstufe und einer digitalen Hauptverarbeitungsstufe (bzw. aus entsprechenden Prozessen) besteht, so lässt sich die Umwandlung vom linearen in den logarithmischem Massstab grundsätzlich zwischen oder innerhalb dieser Prozesse, jedoch vor dem Hauptverarbeitungsprozess durchführen.

De Garnfehler verhältnismässig selten auftreten, wird immer dann, wenn fehlerfreies Garn den Messwandler passiert, der Informationsfluss gleich Null sein und erst dann, wenn ein Fehler auftritt, ansteigen. Dieser Informationsfluss ist aber durch die Erscheinungsform des Garnfehlers begrenzt, da sie verhältnissmässig schnell während des Abspulens durch den Messwandler in Erscheinung treten, dann aber ein relativ langes Beharrungsvermögen aufweisen könne. bis die ebenfalls wieder schnell verschwinden. Der Informationsfluss ist weiterhin dadurch begrenzt, dass die Länge von Garnfehlern subjektiv nur etwa im Logarithmus ihrer Ausdehnung empfunden wird. Je länger also die Länge von Garnfehlern seit deren Beginn durch den Signalverarbeitungsprozess schon beobachtet worden ist, um so gröber darf die Analyse durchgeführt werden, ohne dass am Resultat deswegen ein unzulässiger Fehler bemerkbar würde.

Ein Signalverarbeitungsverfahren, das ständig auf rasches Reagieren eingestellt ist, wie es beim Auftreten eines Fehlers sein muss, ist im stationären oder quasistationären Fall (so lange keine als Garnfehler wahrnehmbare Abweichung eintritt) vom Gesichtpunkt der Signalverarbeitung unterbeansprucht.

Die Digitaltechnik bietet nun mittels adressierbarer Speicher und Methoden der Computertechnik die Möglichkeit, Signalverarbeitungsverfahren für schwankende Beanspruchung derart aufzubauen, dass der ein solches Verfahren ausführende Prozess für eine Beanspruchung dimensioniert werden kann, die kleiner ist als was für dauernde Beanspruchung notwendig wäre. Da im vorliegenden Fall der elektronischen Garnreinigung die Zeit für Spitzenbelastung bezogen auf die gesamte Zeit nur klein ist, lassen sich Prozessoren zur Signalverarbeitung derart bauen, dass sich gegenüber herkömmlicher Technik beträchtliche Vorteile in Bezug auf das Verhältnis Leistung zu Kosten ergeben.

Da sich die Zeit selbst weder komprimieren noch expandieren lässt, wird gemäss der vorliegenden Erfindung zum Lastausgleich bei schwankender Beanspruchung zum Mittel der Pufferung von Daten in Warteschlangen gegriffen.

Anschaulich an einem einfachen Beispiel dargestellt, wird dabei mittels eines ersten Prozesses ein Datenreservoir (Pufferspeicher) laufend mit vom Messwandler anfallenden Signaldaten gespeist, während ein zweiter Prozess das Datenreservoir (Pufferspeicher) laufend durch Abarbeitung wieder entleert.

Der erste Prozess erfüllt bei einer Echtzeit-Messwertvararbeitung die Funktion der Datenerfassung an den Messstellen und einer allenfalls notwendigen Signalaufbereitung (z.B. Synchronisation mit einem nicht mit konstanter Geschwindigkeit ablaufenden Prozess, Nullpunkt- und Driftkorrektur, Linearisierung der Messfühlercharakteristik, A/D-Wandlung, Signalkompression beispielsweise gemäss dem vorstehend genannten Verfahren der Umwandlung der Signaldarstellung vom linearen zum logarithmischen Massstab, etc), und speichert schliesslich die aufbereiteten Messdaten geordnet im Datenreservoir (Pufferspeicher).

Der zweite Prozess entnimmt aus dem vorstehend genannten Datenreservoir (Pufferspeicher) die vorverarbeiteten Messdaten und unterwirft diese der Signalanalyse. Falls es sich, wie hier angenommen, um die Analyse selten auftretender Fehler geht, wird die Signalanalyse in den häufigsten Fällen zu einem negativen Befund kommen. Dies ist jeweils mit wenigen Operationsschritten festzustellen, der Analyseprozess läuft als schnell ab. In selteneren Fällen wird die Fehleranalyse jedoch auf einen positiven Befund stossen, welcher genauer zu analysieren ist. Gegebenenfalls sind aufgrund einer solcher detaillierten Analyse weitere Prozesse auszulösen. Dazu werden im allgemeinen mehrere Operationsschritte benötigt, der Analyseprozess läuft infolgedessen zeitweise langsamer ab.

Mittels der vorstehend genannten Pufferung lässt sich nun eine verbesserter Lastausgleich dadurch erreichen, dass der Prozessor, auf dem der genannte zweite Prozess abläuft, so dimensioniert wird, dass er im Mittel über eine gewisse Zeitspanne mit genügender Sicherheit in der Lage ist, das im Datenreservoir (Pufferspeicher) gelieferte Messdatenmaterial abzuarbeiten. In den selteneren Fällen, wo Fehler zu analysieren sind, wird im Datenreservoir (Pufferspeicher) ein grösserer Rückstau (Warteschlange) auftreten, der während der restlichen Zeit wieder abgebaut wird.

Der statistische Ausgleich bei derartigen Fehleranalyseprozessen lässt sich verbessern, indem mit dem genannten zweiten Prozess mehrere Messstellen zyklisch hintereinander analysiert werden, da in der Praxis statistische Unabhängigkeit bezüglich der auftretenden Fehler zwischen den Messstellen untereinander angenommen werden kann. Oder imgekehrt ist es möglich, aufgrund des erfindungsgemässen Verfahrens mittels eines Prozessors gegebener Leistungsfähigkeit die anfallenden Daten einer grösseren Anzahl Messstellen zu verarbeiten.

Anhand der Beschreibung und der Figuren wird das erfindungsgemässe Verfahren näher erläutert. Dabei zeigt Figur 1 ein Diagramm eines Garnabschnittes mit Toleranzgrenzen in der Querdimension des Garnes, Figur 2 ein Diagramm eines Garnabschnittes mit Bewertungszonen ebenfalls in der Querdimension des Garnes, Figur 3 ein Diagramm eines Garnabschnittes mit Bewertungszonen in der Querdimension und Längsdimension des Garnes, Figur 4 das Prinzip der Prozessverarbeitung und Datenspeicherung für die Auswertung, Figur 5 eine Ausgestaltung eines Uebertragungskanals.

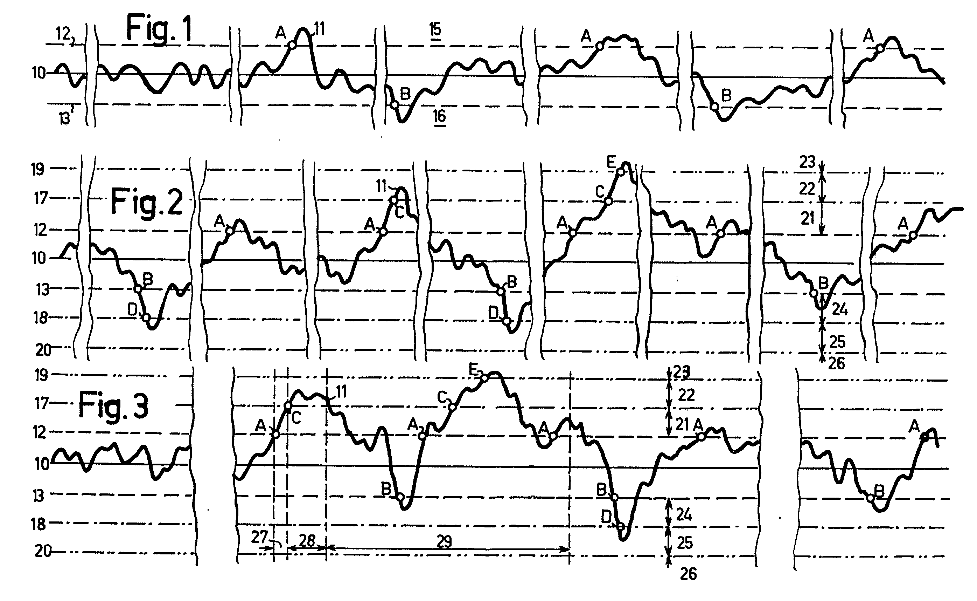

Figur 1 zeigt die an sich bekannte Darstellung des Querschnittes eines Garns als eine um einem Mittelwert 10 variierende Signalkurve 11, wie sie beispielsweise von einem Registriergerät aufgezeichnet wird, an dessen Eingang das von einem den Querschnitt des Garnes abtastenden Messgerät gelieferte elektrische Signal liegt. Weiter zeigt Figur 1 je eine obere Toleranzgrenze 12 und eine untere Toleranzgrenze 13, deren Ueberschreiten durch die Signalkurve 11 ausgewertet werden soll. Sobald die Signalkurve 11 diese Toleranzgrenzen überschritten hat (Punkt A bzw. B), gelangt sie in eines der Gebiete 15, 16.

Die Aussage, dass die Signalkurve 11 vorgegebene Toleranzgrenzen überschreitet, ist für die Belange der Digitaltechnik dahin zu interpretieren, dass die Amplitude der Signalkurve 11 mittels einer vorgegebenen Taktfrequenz abgetastet wird und dabei jeder Amplitudenwert mit dem die Toleranzgrenze darstellenden Wert verglichen wird.

Gemäss der in Figure 2 dargestellten weiteren Ausbildung des Verfahrens werden nun die ausserhalb der Toleranzgrenzen 12, 13 liegenden Gebiete 15, 16 durch weitere Toleranzgrenzen 17, 19 bzw. 18, 20 in Querzonen 21, 22, 23, 24, 25, 26 eingeteilt. Jede dieser Querzonen bildet nun ein Kriterium dafür, dass durch das Eintreten in die nächste Querzone, bzw. Verlassen der vorhergehenden Querzone durch die Signalkurve 11 eine bestimmte Auswirkung der betreffenden relativen Abweichung erwartet wird.

Gemäss der in Figur 3 dargestellten weiteren Ausbildung des Verfahrens wird nun auch die Längsdimensionen des Garnes in Längszonen 27, 28 und 29 eingeteilt. Die Längszone 27 ist beispielzweise dadu.:ch definiert, dass sie jeweils dann beginnt, wenn die Signalkurve 11 die obere Toleranzgrenze 12 oder die untere Toleranzgrenze 13 nach einer fehlerfreien Garnstrecke erstmals wieder überschritten hat (Punkte A, B). Das Signal wird in dieser Längszone 27 mittels des vorstehend genannten zweiten Prozesses auf vorerst kurze Fehler analysiert, indem beispielsweise bei jedem Abtasttakt die Amplitude der Signalkurve 11 auf Ueber- oder Unterschreiten einer der Toleranzgrenzen 12, 17, 19 bzw. 13, 18, 20 analysiert wird. Bleibt der Wert des Signals 11 während mehr als einer bestimmten Anzahl Abtasttakte immer ausserhalb der Grenze 12 bzw. 13, so handelt es sich nicht mehr um einen Fehler kurzer, sondern um einen Fehler mittlerer Längsausdehnung. Die Grenze zwischen Längszone 27 und 28 kann als Grenze zwischen Garnfehlern kurzer und mittlerer Längsausdehnung aufgefasst werden. Sobald sich nun die Signalkurve innerhalb der Längszone 28 und gleichzeitig immer noch ausserhalb entweder der oberen Toleranzgrenze 12 oder der unteren Toleranzgrenze 13 befindet, kann ein gröberes Analysekriterium zur Anwendung kommen, indem beispielsweise nur jeweils jeder zweite oder dritte Abtastwert oder ein Mittelwert von diesen auf Ueberschreiten einer der Toleranzgrenzen 12, 17, 19 bzw. 13, 18, 20 untersucht wird. Damit ist einem Garnfehler mittlerer Längsausdehnung entsprechenden reduzierten Signalverarbeitungsbedürfnis im Sinne einer Aufwandminderung entsprochen. Sinngemäss entspricht eine längszone 29 Garnfehlern grosser Längsausdehnung mit entsprechend nochmals gegenüber Längszone 28 reduziertem Signalverarbeitungsbedürfnis. Hier kann ein nochmals gröberes Analysekriterium zur Anwendung kommen, indem beispielsweise nur jeweils jeder vierte, fünfte oder noch höhere Abtastwert oder ein Mittelwert von diesen auf Ueberschreiten einer der Toleranzgrenzen 12, 17, 19 bzw. 13, 18, 20 untersucht wird. Damit ist einem für Garnfehler grösserer Längsausdehnung nochmals reduzierten Signalverarbeitungsbedürfnis im Sinne einer Aufwandminderung erneut entsprochen.

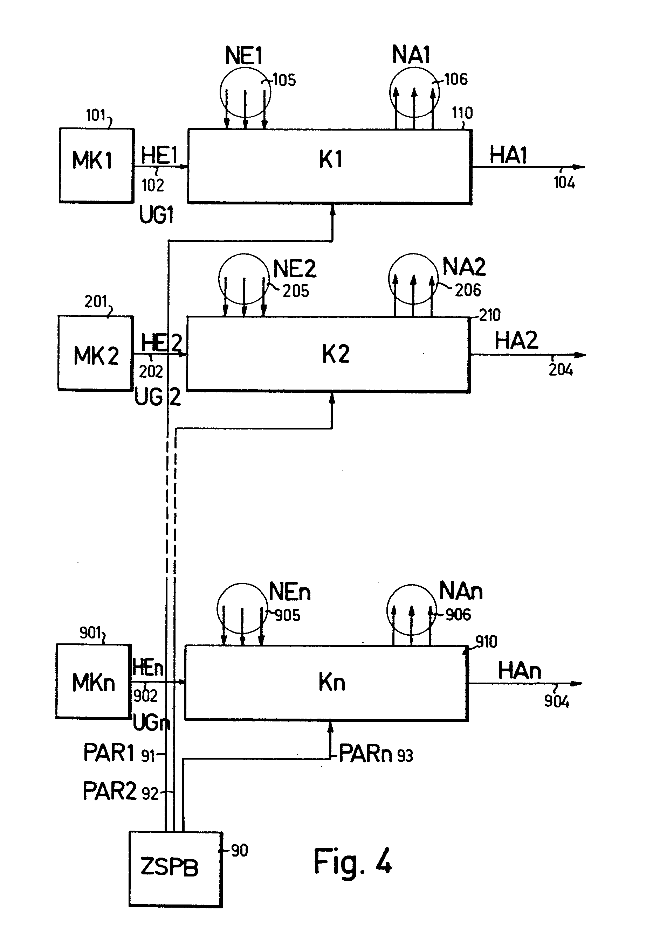

Anhand von Fig. 4 wird eine mögliche Anwendung des erfindungsgemässen Verfahrens am Prinzipschema einer Vorrichtung zur elektronischen Reinigung von Garnen und Klassierung von Garnfehlern dargestellt.

Die Vorrichtung besteht aus n Kanälen K1 110, K2 210, Kn 910, von denen jeder einer n Spullstellen umfassenden Sektion einer Spulmaschine zugeordnet ist. Jeder dieser Kanäle wird an seinem Haupteingang HE1 102, HE2 202, HEn 902, von einem Messkopf MK1 101, MK2 201, MKn 901, mit einem Garnmessfühler gespeist, welcher eine Spannung UG1, UG2, UGn abgibt, deren Wert jederzeit ein Mass für den Querschnitt (oder Durchmesser) des laufend abgetasteten Garns ist. Jeder dieser Kanäle besitzt ferner einen Hauptausgang HA1 104, HA2 204, HAn 904, an welchem ein Signal erscheint, das zur Steuerung einer Garntrennvorrichtung nach bekannter Tecknik verwendet werden kann. Jeder Kanal besitzt ferner Nebeneingänge NE1 105, NE2 205, NEn 905 (für externe Schnittbefehle, Lauf/Stop-Signal etc.) sowie eine Anzahl Nebenausgänge NA1 106, NA2 205, NAn 906 für statistische Daten über die Produktion der entsprechenden Spulstelle (Garnschnittstatistiken, Garnfehlerstatistiken, Stillstandszeiten der Spulstelle etc.), oder davon abgeleitete Steuerungs- oder Warnfunktionen.

Alle Kanäle haben ferner Zugriff zu einem zentralen Speicherbereich ZSPB 90, wo die diversen zur Signalanalyse bzw. zur Auslösung von Steurungs- und Warnfunktionen notwendigen Parameter PAR1 91, PAR2 92, PARn 99 gespeichert sind (Toleranzgrenzen für Quer- und Längsdimension, Fehlerbewertungskriterien, Steuer- und Warnkriterien etc., eventuell Logarithmustabelle, Garnnummer, Materialkennziffer etc.).

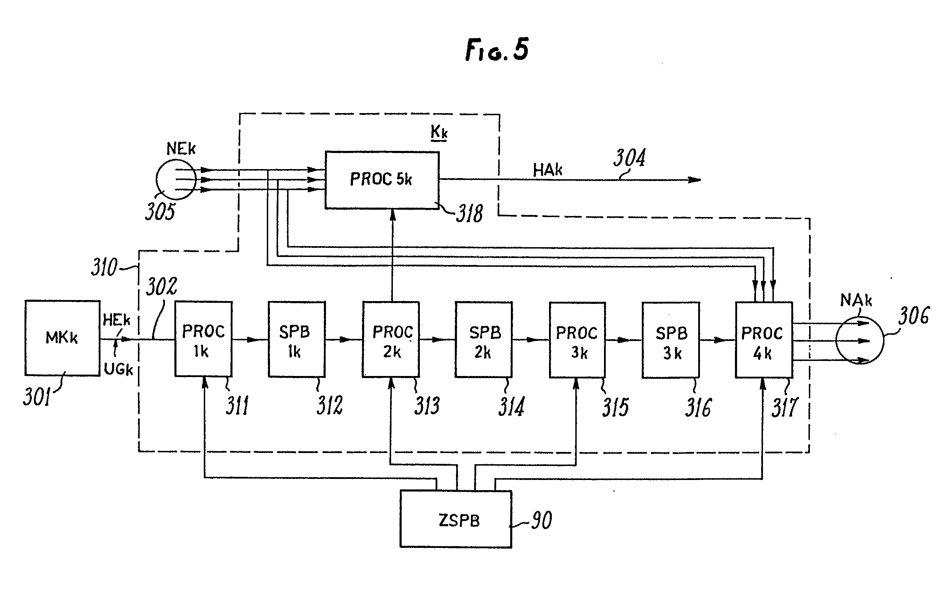

In Figur 5 ist ein solcher Kanal einzeln dargestellt. Der k-te Messkopf MKk 301 ist mit einem ersten Prozess PROC1k 311 verbunden, dieser wiederum mit einem ersten Speicherbereich SPB1k 312, dieser wiederum mit einem zweiten Prozess PROC2k 313, dieser wieder mit einem zweiten Speicherbereich SPB2k 314, dieser wieder mit einem dritten Prozess PROC3k 315, dieser wieder mit einem dritten Speicherbereich SPB3k 316, dieser schliesslich mit einem vierten Prozess PROC4k 317. An diesen vierten Prozess PROC4k 317 sind die Nebenausgänge NAk 305 angeschlossen. Ferner besteht eine direkte Verbindung vom zweiten Prozess PROC2k 313 zum Schnittauslöseprozess PROC5k 318. Der Ausgang des Schnittauslöseprozesses PROC5k 318 ist der Hauptausgang HAk 304, also das Schnittauslösesignal. Die Nebeneingänge NEk 305 führen sowohl zum Schnittauslöseprozess PROC5k 318 als auch zum vierten Prozess PROC4k 317. Ferner haben alle Prozesse Zugriff zum zentralen Speicherbereich ZSPB 90. Die Nebeneingänge NEk 305 führen parallel zum Schnittauslöseprozess PROC5k 318 und zum vierten Prozess PROC4k 317, die Nebenausgänge NAk 306 entspringen dem vierten Prozess PROC4k 317.

Die Funktion der in Fig. 4 dargestellten Vorrichtung sei im folgenden am k-ten Kanal (Fig. 5) näher erläutert.

Der Messkopf MKk 301 gibt eine zeitlich variable Gleichspannung UGk ab, welche proportional zum jeweiligen Querschnitt (Durchmesser) des durch den Messkopf MKk 301 laufenden Garnes ist. Im ersten Prozess PROC1k 311 wird diese Gleichspannung zeitdiskret abgetastet und in 16 bit-Worte digitalisiert. Ferner wird im ersten Prozess PROC1k 311 mittels einer im zentralen Speicherbereich ZSPB 90 abgespeicherten Logarithmentabelle (table-look-up Methode) das digitalisierte Garnsignal vom linearen Massstab in den logarithmischen Massstab umgewandelt und gleichzeitig die Wortlänge von 16 bit auf 8 bit reduziert. Eine alternative Lösung bestünde darin, dass die Umwandlung vom linearen in den logarithmischen Massstab gleichzeitig mit der Analog-Digital-Umwandlung stattfindet, beispielsweise unter sinngemässer Anwendung von in der Telefonietechnik (Pulsecodemodulation) bekannt gewordenen sogenannten CODEC-Schaltungen und unter Verwendung von speziell für diesen zweck erhältlichen integrierten Schaltelementen. Ferner wird im ersten Prozess PROC1k 311 die Zeitabhängigkeit des digitalisierten Garnsignals eliminiert, indem die Taktfrequenz der Analog/Digitalumwandlung proportional zur Geschwindigkeit des sich durch den Messkopf bewegenden Garnes gemacht wird. Diese geschieht gemäss Fig. 5 unter Verwendung des im zentralen Speicherbereich ZSPB 90 abgespeicherten Sollwertes der für die betreffende Spulstelle verwendeten Spulgeschwindigkeit oder alternativ in bekannter Weise durch eine (in Fig. 5 nicht gezeigte) direkte Synchronisation mit der Drehgeschwindigkeit der auf der Spulstelle angeordneten Nutentrommel. Ferner wird im ersten Prozess PROC1k 311 eine Normalisierung des Garnsignals auf konstanten Nennquerschnitt (Durchmesser) und konstante (dielektrische) Garneigenschaften vorgenommen. Die entsprechenden Werte (Garnnummer, Materialziffer) werden dem zentralen Speicherbereich ZSPB 90 entnommen. Da sich diese Werte multiplikativ auf das dem Garnquerschnitt (Durchmesser) proportionale Signal auswirken, läuft ihre Berücksichtigung im Garnsignal, welches im logarithmischen Mass dargestellt ist, auf einfache Addition bzw. Subtraktion heraus. Dies bedeutet zusätzlich zur reduzierten Wortlänge eine beträchtliche Vereinfachung durch die erfindungsgemässe Darstellung des Garnsignals im logarithmischen Massstab. Nach all diesen Umwandlungen wird vom ersten Prozess PROC1k 311 eine Folge von normierten Werten in den ersten Speicherbereich SPB1k 312 abgelegt, wobei von jedem Wert zum nächsten sich das abgetastete Garn unabhängig von der Spulgeschwindigkeit um eine konstante Weglänge weiterbewegt hat. Die im ersten Speicherbereich SPB1k 312 abgelegten Werte sind also auf einen Querschnitt (Durchmesser) "eins" normiert und nunmehr ausschliesslich vom relativen Durchmesser des Garns und von der gespulten Weglänge abhängig.

Die Speicherbereiche SPB1k 312, SPB2k 314, SPB3k 316 sind gemäss Fig. 5 alle hardwaremässig implementierte sogenannte FIFO-Speicher (first-in, first-out). Andere, Softwareorientierte Speichermethoden sind im Rahmen des erfindungsgemässen Verfahrens ebenfalls denkbar, wobei die Speicherplatzverwaltung durch einen in Fig. 4 und Fig. 5 nicht gezeigten Speicherplatzverwaltungsprozess vorzunehmen wäre.

In einem zweiten Prozess PROC2k 313 werden aus dem ersten Speicherbereich SPB1k 312 laufend Werte entnommen und einem Signalanalyseprozess unterzogen. Dieser Signalanalyseprozess besteht erfindungsgemäss darin, dass jeder einzelne der normierten Querschnittswerte daraufhin untersucht wird, ob er eine der Fehlertoleranzgrenzen, welche im zentralen Speicherbereich ZSPB 90 abgespeichert sind, über- oder unterschritten hat, oder ob er in derselben Toleranzzone wie sein Vorgänger liegt. Im häufigsten Fall wiid die relative Garnabweichung innerhalb der untersten Fehlertoleranzgrenze liegen, so dass keien weitere Untersuchung dieses Wertes mehr nötig ist. Nur wenn eine der genannten Toleranzgrenzen über- bzw. unterschritten wird, ist eine eingehendere Analyse gerechtfertigt. Je nachdem, wie lange das Signal schon in einer Fehlerzone beharrt und in welcher Fehlerzone sich den Signalwert gerade befindet, kann ein potentiell zu reinigender Garnfehler vorliegen. Ist das der Fall, benachrichtigt der zweite Prozess PROC2k 313 den Schnittauslöseprozess PROC5k 318. Dieser untersucht dann unabhängig vom zweiten Prozess PROC 2k 313, ob auf Grund der an den Nebeneingängen NEk 305 liegenden Signale ein Garnschnitt gerechtfertigt ist oder nicht. Zutreffendenfalls wird vom Schnittauslöseprozess PROC5k 318 an den Hauptausgang HAk 304 ein Schneidbefehl abgegeben.

Für die Zwecke der Garnfehlerklassierung (geschnittene und nicht geschittene Garnfehler) muss das Garnsignal nach feineren Kriterien analysiert werden, als dies für die Garnfehlereinigung notwendig ist. Es genügt jedoch, wie vorstehend erwähnt, wenn festgestellt wird, wenn (bezogen auf die seit dem jeweilig erstmaligen Auftreten des betreffenden Fehlers) die Garnabweichung in eine neue Toleranzzone eingetreten ist oder eine solche verlassen hat. Dieses Eintreten/Austreten in eine neue Toleranzzone wird ebenfalls vom zweiten Prozess PROC 2k 313 überwacht. Jedesmal, wenn ein solches verhältnismässig seltenes Ereignis eingetreten ist, wird dies unter Angabe, was geschehen ist und bei welcher Garnlänge dies eingetreten ist, im zweiten Speicherbereich SPB2k 314 in geeignet codierter Form protokolliert. Der zweite Speicherbereich SPB2k 314 wird also laufend mit einem oder mehreren Datenworten gespeist, welche solche Zustandsänderungen, sowie den Ort auf dem gespulten Garn, wo sie aufgetreten sind, enthalten.

In einem dritten Prozess PROC3k 315 wird aus diesen Werten in bekannter Art eine Garnfehlerklassierung, beispielsweise nach einem Tabellenschema vorgenommen (Uster Classimat Prinzip). Die für diese Klassierung notwendigen Parameter werden, sofern sie nicht schon im zweiten Prozess PROC2k 313 verwendet wurden, aus dem zentralen Speicherbereich ZSPB 90 entnommen. Die fertig klassierten Garnfehler werden vom Prozess PROC3k 315 unter Angabe eines Ortes, wo sie auf dem Garn aufgetreten sind, in geeigneter Form codiert in einen dritten Speicherbereich SPB3k 316 abgespeichert.

In einem vierten Prozess PROC4k 317 werden nun diese im dritten Speicherbereich SPB3k 316 abgelegten klassierten Garnfehler zusammen mit den via Nebeneingänge NEk 305 eingegebenen Zustandsangaben über das Verhalten der Spulstelle und des Reinigers in bekannter Art und Weise zu Meldungen zusammengefasst und weiter verarbeitet.

Zusammenfassend kann festgestellt werden, dass in einer Vorrichtung gemäss Fig. 4 und 5 für jeden Kanal fünf asynchron und durch als Puffer wirkende Speicherbereiche voneinander entkoppellte Prozesse ablaufen, die sich dadurch auszeichnen, dass sie durch einen durch die subjektive Wahrnehmung bedingten minimalen technischen Aufwand realisiert werden können.

Aufgrund des erfindungsgemässen Verfahrens wird der Fachmann in der Praxis gewisse Prozesse, je nach Anwendungen und Verhältnissen, auf einem oder mehreren gemeinsamen Prozessoren ablaufen lassen. Unter Annahme einer aus n Kanälen gemäss Fig. 4 und 5 gebauten Vorrichtung wird es in gewissen Fällen vorteilhaft sein, beispielsweise die gleichartigen Prozesse PROC11, PROC1k bis PROC1n auf einem speziell dafür vorgesehenen Prozessor und die restlichen gleichartigen Prozesse PROC31, PROC3k ..., PrOC3n sowie PROC41, PROC4k ..., PROC4n, sowie PROC51, PROC5k ..., PROC5n auf einem jeweils weiteren gemeinsamen Prozessor zu implementieren.

Für die ersten zwei Gruppen könnte es vorteilhaft sein, spezielle Prozessoren mit Komponenten der sogenannten Bit-Slice-Technik zu bauen und für die Prozesse der letzteren Gruppen einen universellen Mikroprozessor oder Mikrocomputer anzuwenden.

Ohne vom erfindungsgemässen Verfahren abzuweichen, kann es jedoch auch vorteilhaft sein, sämtliche Prozesse wenigstens eines Kanals auf einem gemeinsamen Prozessor zu implementieren oder gewisse Teile in herkömmlicher digitaler Bauweise auszuführen. Es ist für den Fachmann ebenfalls selbstverständlich, dass sich die als hardwaremässige FIFO Pufferspeicher darstellbaren diversen Speicherbereich auch auf einem oder mehreren adressierbaren Speichern unterbringen lasse wobei—wie schon vorstehend angedeutet—ein spezieller (gemeinsamer), in Fig. 4 und 5 nicht gezeigter Prozess zur Speicherplatzverwaltung vorzusehen ist.

**Patentansprüche**

1. Verfahren zur Festellung und Auswertung von Fehlern in Garnen, bei dem:

— ein die Dicke des Garns als Funktion des Ortes repräsentierendes Messsignal erzeugt wird,
— diskrete aufeinanderfolgende Werte des Messsignals in logarithmierte und digitalisierte Zahlenwerte umgesetzt werden,
— die Zahlenwerte laufend mit Dickentoleranzen darstellenden Schwellwerten verglichen werden,
— die Zahlenwerte vor dem Vergleich zwischengespeichert werden und die Auswertung anhand dieser zwischengespeicherten Zahlenwerte entkoppelt von der Speicherung dieser Werte durchgeführt wird, indem:

a) die Länge des Garnabschnitts, über die eine Uberschreitung der Schwellwerte durch aufeinanderfolgend abgespeicherte Zahlenwerte stattfindet, mit vorgegebenen Sektionen unterschiedlicher Längen verglichen wird, und,
b) die Anzahl der zur Auswertung des Fehlers eines fehlerbehafteten Garnabschnitts herangezogenen zwischengespeicherten Zahlenwerte diese Abschnitts in Abhängigkeit vom Ergebnis des Vergleichs der Länge des fehlerbehalteten Garnabschnitts mit der Länge der vorgegebenen Sektionen gewählt wird, wobei die ausgewertete Anzahl von Zahlenwerten mit zunehmender Länge des Garnabschnitts reduziert wird.

2. Verfahren nach Anspruch 1, dadurch gekennzeichnet, dass die Breite des Toleranzbereiches eine Funktion der statistischen Ungleichmässigkeiten des Garnsignals ist.

3. Verfahren nach einem der Ansprüche 1 oder 2, dadurch gekennzeichnet, dass die spezifische Fehleranalyse in eine bestimmte Anzahl von Teilprozessen aufgeteilt wird, welche Teilprozesse mittels Pufferspeichern zeitlich entkoppelt werden, sowie dadurch, dass die selteneren und zeitlich weniger kritischen Teilanalysen in den nachgelagerten Teilprozessen vorgenommen werden.

4. Verfahren nach Anspruch 1, dadurch gekennzeichnet, dass die Logarithmierung des Messsignals vor der digitalen Umsetzung erfolgt.

5. Verfahren nach Anspruch 4, dadurch gekennzeichnet, dass die Logarithmierung des Messsignals mit Mitteln der Analogtechnik durchgeführt wird.

6. Verfahren nach Anspruch 4, dadurch gekennzeichnet, dass die Logarithmierung des Messsignals mittels eines Analog-Digitalwandlers mit logarithmierendem Ausgang durchgeführt wird.

7. Verfahren nach Anspruch 1, dadurch gekennzeichnet, dass die Logarithmierung mit Mitteln der Digitaltechnik durchgeführt wird.

## Revendications

1. Procédé pour déterminer et évaluer des défauts dans des fils, qui consiste à:

— produire un signal de mesure qui représente l'épaisseur des fils en tant qu'une fonction de leur longueur;
— transformer les valeurs discrètes successives du signal de mesure en données logarithmiques digitales;
— comparer en continu lesdites données à des valeurs de seuil représentant les tolérances de l'épaisseur;
— emmagaziner les données avant leur comparaison, et exécuter l'évaluation desdites données emmagasinées, découplées en temps de l'instant de leur emmagasinage,

dans lequel,
a) on compare la longueur de la section du fil qui montre un dépassement des valeurs de seuil par les données emmagasinées successives à des sections prédéterminées de différentes longueurs; et,
b) on sélectionne un tel nombre de données emmagasinées dont on tient compte pour évaluer les défauts en fonction du résultat de la comparaison entre la longueur de la section de fil défectueux et la longueur des sections prédéterminées, afin que ce dit nombre de données évaluées soit diminué par rapport à la longueur lorsque augmente la longueur de la section de fil.

2. Procédé selon la revendication 1, caractérisé en ce que la largeur de la plage de tolérance est fonction des irrégularités statistiques du signal de mesure.

3. Procédé selon l'une des revendications 1 ou 2, caractérisé en ce que l'analyse spécifique des défauts est décomposée en un certain nombre de processus partiels, désaccouplés dans le temps per des mémoires tampons, et en ce que des analyses partielles plus rares et moins critiques dans le temps sont réalisées dans les processus partiels successifs.

4. Procédé selon la revendication 1, caractérisé en ce que la transformation du signal de mesure en données logarithmiques s'effectue avant la transformation digitale.

5. Procédé selon la revendication 4, caractérisé en ce que la transformation logarithmique du signal de mesure est obtenue à l'aide de la technique analogique.

6. Procédé selon la revendication 4, caractérisé en ce que la transformation logarithmique du signal de mesure est obtenue par un convertisseur analogique-digital à sortie logarithmique.

7. Procédé selon la revendication 1, caractérisé en ce que la transformation logarithmique du signal de mesure est obtenue à l'aide de moyens relevant de la technique digitale.

## Claims

1. A process for determining and evaluating faults in yarns, comprising:

— producing a measuring signal which represents the thickness of the yarns as a function of their length;
— transforming consecutively sampled discrete values of the measuring signal into logarithmic and digitalized data samples;
— comparing said data samples continuously with threshold values representing thickness tolerances;
— storing the data samples before the comparison and executing the evaluation of said stored data samples isolated with respect to the time at which said data samples have been stored,

wherein,
a) the length of the yarn section at which the thickness of the yarn exceeds the threshold values on consecutive samples of the stored data is compared with predetermined sections of different lengths, and,
b) a number of stored data samples, which are considered for evaluating the fault in dependence of the result of the comparison between the length of the faulty yarn section and the length of the predetermined section is selected in order for said number of data samples to be decreased per unit of length with increasing length of the yarn section.

2. Process according to Claim 1, characterized in that the size of the tolerance band is a function of the statistical irregularity of the measuring signal.

3. Process according to any one of Claim 1 or 2, characterized in that the specific fault analysis is distributed over a number of sub-processes, which sub-processes are isolated with respect to time by buffer stores, and in that the more rare and temporally less critical analyses are executed in the subsequent subprocesses.

4. Process according to Claim 1, characterized in that the production of the logarithmic measuring signal takes place before the transformation into digital data.

5. Process according to Claim 4, characterized in that the production of the logarithmic measuring signal is carried out by means of analog technique.

6. Process according to Claim 4, characterized in that the production of the logarithmic measuring signal is carried out by means of an analog-digital converter with logarithmic output.

7. Process according to Claim 1, characterized in that the production of the logarithmic measuring signal is carried out by means of digital technique.

Fig.1

Fig.2

Fig.3

0 025 035

Fig. 4

Fig.5